# EUROPEAN PATENT SPECIFICATION

(11) **EP 1 901 712 B1**
(45) Date of publication and mention of the grant of the patent: **20.06.2012**
(21) Application number: 06743894.5
(22) Date of filing: 12.05.2006
(51) Int. Cl.: A61K 31/08, A61K 9/12, A61P 9/10, A61P 25/28

(54) **Therapeutic sclerosing foam made preferably from xenon**
Therapeutischer sklerosierender Schaum vorzugsweise aus Xenon
Mousse thérapeutique sclérosante fabriquée préférablement à partir du xénon

(30) Priority: 13.05.2005 GB 0509824; 24.08.2005 GB 0517361
(43) Date of publication of application: 26.03.2008
(73) Proprietor: BTG International Limited, London EC4M 7RD (GB)
(72) Inventor: JENKINS, William, John, BTG International Limited, London EC4M 7SB (GB)
(74) Representative: Simpson, Paul Christopher
(86) International application number: PCT/GB2006/001754
(87) International publication number: WO 2006/120469

(56) References cited:
- WO-A-02/41872
- WO-A-2004/062461
- WO-A-2005/048976
- WO-A-2005/048984
- DE-A1- 19 933 704
- US-B1- 6 572 873

## Description

The present invention relates to a therapeutic foam e.g. for treatment of varicose veins.

In recent years the use of sclerosing chemicals in the form of foams has become a popular way of treating varicose veins. Foam is injected into the affected vein whilst it progress is monitored using ultrasound scanning. The process causes sclerosis and functional elimination of the vein. Other disorders such as venous malformations may also be treated this way.

Most practitioners make foam extemporaneously using ambient air as the gaseous component. As extensively discussed in copending patent applications nos. PCT/GB04/004824, PCT/GB041004831, PCT/GB04/004846 and PCT/GB04/004848, the use of air foams is potentially harmful and, in the view of the inventors, is to be avoided. The inventors have followed the key disclosure by the Cabreras in their patent EP0656203B who first proposed a microfoam with oxygen or carbon dioxide as the gas component. Carbon dioxide and oxygen are respectively dissolved blood in or absorbed by haemoglobin, thus making both considerably more attractive than air.

There are other gases which are very soluble, e.g. nitrous oxide. Helium has also been considered since it passes easily across pulmonary gas exchange membranes and hence is exhaled from the body quickly.

The inventors have now surprisingly realised that the gas need not be soluble and need not necessarily be absorbed only by the blood: it is possible to overcome the problem of foam treatment leaving residual gas in the body by using a gas component which dissolves into tissue other than blood, e.g. the vein walls. In this context, they have conceived of the possibility of using a lipid soluble gas, e.g. one or more of the noble gases, as a substantial proportion (greater than 40%) of the gas component of the foam. The higher molecular weight noble gases, especially xenon, are known to dissolve in lipid, e.g. in cell walls. Thus it appears that these gases may be taken up by the body quickly if injected into the venous system, both into the cells of the vein walls and into the blood cells. This dual mode of uptake may give rise to very fast absorption, especially if the gas also has a degree of solubility in water as well. Speed of uptake is critical because if gas is taken up slowly, this may give the opportunity for nitrogen which is already dissolved in the blood to diffuse into the foam, potentially causing enduring bubbles. Again, this is discussed at length in the copending applications mentioned above.

US-B 1-6 572 873, WO 02/41872, WO 2004/062461, WO 2005/048976 and WO 2005/048984 do not describe, nor do they suggest, a foam containing xenon as described in present claim 1.

According to the present invention a foam is provided comprising a liquid phase and a gas phase wherein the liquid phase comprises at least one sclerosing agent and the gas phase comprises between 41% and 100% of a gas which is lipid soluble, the lipid soluble gas being argon, krypton or xenon or a mixture of two or more of these gases. Preferably the gas phase comprises between 50 and 100%, more preferably between 60 and 100%, still more preferably between 70 and 100%, 80 and 100% or 90 and 100% of the said lipid soluble gas.

The most preferred lipid soluble gas is xenon. Xenon is used as an anaesthetic, and its behaviour in body tissue is well understood: see for example "Xenon Anesthesia": Lynch et al, Anesthesiology, v92, No.3, March 2000*.* Xenon has the further advantage that it is moderately soluble in water (mole fraction solubility 7.9 x 10⁻⁵, which is three times as soluble as oxygen).

If the lipid soluble gas does not make up 100% of the gas phase, then it is preferred that the remainder of the gas phase consist essentially of oxygen, carbon dioxide or a mixture of the two.

### Example

A fume cupboard is filled with greater than 99% pure xenon gas, after placing in the cupboard a small (100ml) beaker containing 10ml 1% polidocanol solution, together with a small hand held electric motor with a chuck in which is mounted a small (10mm diameter) dental brush. The motor is switched on and the speed adjusted to 12,000 r.p.m. The dental brush is then gently inserted into the liquid in the beaker, such that a vortex is formed in the surface of the liquid in the beaker. After a few seconds a foam forms, which becomes finer and more homogeneous as time goes on. After 90 seconds the brush is removed. In the beaker is a stiff, homogeneous foam whose bubbles are in the main part invisible to the naked eye (a "microfoam"). The beaker may be inverted for a few seconds without the foam falling out. 30ml of the foam is then drawn out of the beaker using a syringe for use in the treatment of a human patient's varicose veins using methodology which is generally known in this field.

## Claims

1. A foam having a liquid phase and a gas phase wherein the liquid phase comprises at least one sclerosing agent and the gas phase comprises between 41% and 100% of a gas which is lipid soluble, the lipid soluble gas being argon, krypton or xenon or a mixture of two or more of these gases.

2. A foam as claimed in claim 1 wherein the gas phase comprises between 50 and 100%, more preferably between 60 and 100%, still more preferably between 70 and 100%, 80 and 100% or 90 and 100% of the said lipid soluble gas.

3. A foam as claimed in any preceding claim wherein the remainder of the gas phase is substantially made up by oxygen, carbon dioxide or a mixture of oxygen and carbon dioxide.

4. A foam as claimed in any preceding claim wherein the sclerosing agent is polidocanol solution.

## Patentansprüche

1. Schaum mit einer flüssigen Phase und einer Gasphase, wobei die flüssige Phase mindestens ein sklerosierendes Mittel umfasst und die Gasphase zwischen 41% und 100% eines Gases, das lipidlöslich ist, umfasst, wobei das lipidlösliche Gas Argon, Krypton oder Xenon oder eine Mischung von zwei oder mehr dieser Gase ist.

2. Schaum, wie in Anspruch 1 beansprucht, wobei die Gasphase zwischen 50 und 100%, stärker bevorzugt zwischen 60 und 100%, noch stärker bevorzugt zwischen 70 und 100%, 80 und 100% oder 90 und 100% des lipidlöslichen Gases umfasst.

3. Schaum, wie in einem beliebigen der vorhergehenden Ansprüche beansprucht, wobei der Rest der Gasphase im Wesentlichen aus Sauerstoff, Kohlenstoffdioxid oder einer Mischung von Sauerstoff und Kohlenstoffdioxid besteht.

4. Schaum, wie in einem beliebigen der vorhergehenden Ansprüche beansprucht, wobei das sklerosierende Mittel eine Polydocanol-Lösung ist.

## Revendications

1. Mousse présentant une phase liquide et une phase gazeuse dans laquelle la phase liquide comprend au moins un agent sclérosant et la phase gazeuse comprend entre 41 % et 100 % d'un gaz qui est soluble dans un lipide, le gaz soluble dans un lipide étant l'argon, le krypton ou le xénon ou un mélange de deux ou plus de ces gaz.

2. Mousse telle que revendiquée dans la revendication 1 dans laquelle la phase gazeuse comprend entre 50 et 100 %, plus avantageusement entre 60 et 100 %, encore plus avantageusement entre 70 et 100 %, 80 et 100 % ou 90 et 100 % dudit gaz soluble dans un lipide.

3. Mousse telle que revendiquée dans l'une quelconque des revendications précédentes dans laquelle le reste de la phase gazeuse est substantiellement constitué d'oxygène, 1 de dioxyde de carbone ou d'un mélange d'oxygène et de dioxyde de carbone.

4. Mousse telle que revendiquée dans l'une quelconque des revendications précédentes dans laquelle l'agent sclérosant est une solution de polidocanol.
